# EUROPEAN PATENT APPLICATION

(11) **EP 2 511 363 A1**
(43) Date of publication of application: **17.10.2012**
(21) Application number: 10835879.7
(22) Date of filing: 01.12.2010
(51) Int. Cl.: C11D 17/00, A61K 8/73, A61Q 5/02, A61Q 19/10, C11D 3/37

(54) **CLEANSER COMPOSITION, METHOD FOR GENERATING FOAM, FOAM, AND METHOD FOR WASHING HAIR**

(30) Priority: 08.12.2009 JP 2009278766; 10.12.2009 JP 2009280414
(71) Applicant: Shiseido Company, Ltd., Chuo-ku Tokyo 104-8010 (JP)
(72) Inventor: KAWASOE, Tomoyuki, Yokohama-shi Kanagawa 236-8643 (JP); TERAMOTO, Hiromi, Yokohama-shi Kanagawa 236-8643 (JP)
(74) Representative: TER MEER - STEINMEISTER & PARTNER GbR
(86) International application number: PCT/JP2010/071496
(87) International publication number: WO 2011/070958

(57) **Abstract**

A cleansing composition including water and a surfactant, the content of the surfactant in the cleansing composition being more than or equal to 0.4% by weight and less than or equal to 12% by weight, the viscosity of the cleansing composition at 30°C being more than or equal to 5 mPa·s and less than or equal to 1500 mpa·s, the foam viscosity of foam at 30°C at 10 seconds after the foam is generated by mixing the cleansing composition with air being more than or equal to 40 mPa·s and less than or equal to 100 mPa·s, and the average diameter of bubbles included in the foam at 30 seconds after the foam is generated by mixing the cleansing composition with air being more than or equal to 10µm and less than or equal to 100µm.

## Description

### TECHNICAL FIELD

The present invention relates to a cleansing composition, a method of generating foam, foam, and a method of cleansing hair.

### BACKGROUND ART

Conventionally, for a cleansing agent for cleansing hair, skin or the like, a solid cleansing agent, a liquid cleansing agent or the like are known. Further, a foam cleansing agent has been used as it is easy to lather.

Japanese Laid-open Patent Publication No. 2007-161906 discloses a composition for foam cleansing agent including an anionic surfactant, a fatty acid alkylol amide and a poly(alkylene terephthalate). Further, a foam cleansing agent where the composition for foam cleansing agent is filled in a foam discharging container of a non-aerosol type such as a pump foamer, a squeeze foamer or the like is disclosed.

However, as the content of the surfactant in the composition for foam cleansing agent is large, there is a problem that a discharged amount of the surfactant used when cleansing also becomes large. On the other hand, if the content of the surfactant in the composition for foam cleansing agent is decreased, there is a problem that detergent quality (detergency) and sensation are lowered.

Generally, when using a cleansing agent for hair, skin or the like such as a shampoo, a hand soap, a body shampoo or the like, a user does not use the cleansing agent in a liquid form as it is, but instead the user takes the cleansing agent in the liquid form on one's hand and lathers the cleansing agent on one's palm or hair, or the user uses a container having a lathering function or a lathering apparatus to lather the cleansing agent into foam. Then, the user cleanses one's hair, hand and skin by the cleansing agent in a foam form (foam cleansing agent) to enjoy an appropriate cleansing power.

When using the liquid cleansing agent, it is necessary to lather the liquid cleansing agent before starting a cleansing process, and there is a problem that this process is troublesome. Further, a method of lathering depends on individuals so that a predetermined cleansing effect may not be obtained. However, a user can immediately use a foam cleansing agent by having a container or the like having a lathering function previously generate the foam cleansing agent and obtaining the foam cleansing agent from the container or the like to improve usability and equalize detergency.

Conventionally, for a method of generating a foam cleansing agent from an original liquid cleansing agent, a method of generating foam by a pump foamer, a method of generating foam by aerosol, an electric method of generating foam or the like are known. For the method of generating foam by the pump foamer, a dispenser in which a lathering unit such as a mesh member or the like is provided to generate the foam cleansing agent from the liquid cleansing agent (Japanese Laid-open Patent Publication No. 2008-265877 and Japanese Laid-open Patent Publication No. 2005-262202).

For the method of generating foam by an aerosol container, liquid gas which functions as a propellant is discharged from a nozzle with a liquid cleansing agent to expand drastically to lather the liquid cleansing agent to generate a foam cleansing agent (Japanese Laid-open Patent Publication No. 2009-120525). Further, for the electric method of generating foam, an expanded stone (porous material) which is configured to supply air from a pump into a liquid cleansing agent is provided in a tank filled with the liquid cleansing agent. When fine air is mixed into the liquid cleansing agent by bubbles injected from the expanded stone, the foam cleansing agent is generated (Japanese Laid-open Patent Publication No. 2003-033292). Further, there is a method in which a method of generating foam made practicable in public lavatories and the electric method of generating foam are combined (Japanese Laid-open Utility Model Publication No. H05-007334).

However, for the method of generating foam by the pump foamer, there is a problem that desired creamy foam cannot be generated as the generated foam cleansing agent is not sufficiently fine. For such foam which is not fine, air (bubbles) included in the foam are large so that the foam is easily broken while cleansing, which results in problems that the persistency of the foam is not good and detergency is not satisfied. Further, for the method of generating foam by the pump foamer, there is a problem that the mesh member provided in the dispenser is blocked by the residue and drying of the original liquid cleansing agent along a time so that there is a limitation in the kinds of cleansing agents to be used and the lifetime of the dispenser becomes short, depending on how the dispenser is to be used. Here, although it can be considered that the mesh member is exchanged, generally, as the mesh member is placed backward in the dispenser, it is troublesome and is not realistic to exchange the mesh member.

Further, for the method of generating foam by the aerosol container, liquid gas is necessary as the propellant. Generally, the liquid gas is flammable and may cause a safety problem.

Further, for the conventional electric method of generating foam, as the foam cleansing agent generated by bubbles injected from the expanded stone is not discharged outside until the foam cleansing agent fills the tank, there is a problem that a longer time is necessary to obtain the foam cleansing agent. Further, the foam cleansing agent generated by using the expanded stone is not fine, so that desired creamy foam cannot be generated.

For the method in which the method of generating foam by the pump foamer and the electric method of generating foam are combined, although the blocking of the mesh member is expected to be reduced, the foam cleansing agent is not fine and desired creamy foam cannot be obtained in this case as well. Further, the device becomes large.

### [Patent Document]

[Patent Document 1] Japanese Laid-open Patent Publication No. 2007-161906
[Patent Document 2] Japanese Laid-open Patent Publication No. 2008-265877
[Patent Document 3] Japanese Laid-open Patent Publication No. 2005-262202
[Patent Document 4] Japanese Laid-open Patent Publication No. 2009-120525
[Patent Document 5] Japanese Laid-open Patent Publication No. 2003-033292
[Patent Document 6] Japanese Laid-open Utility Model Publication No. H05-007334

### SUMMARY OF THE INVENTION

According to an embodiment, there is provided a cleansing composition including water; and a surfactant, the content of the surfactant in the cleansing composition being more than or equal to 0.4% by weight and less than or equal to 12% by weight, the viscosity of the cleansing composition at 30°C being more than or equal to 5 mPa•s (sec) and less than or equal to 1500 mPa•s, the foam viscosity of foam at 30°C at 10 seconds after the foam is generated by mixing the cleansing composition with air being more than or equal to 40 mPa•s and less than or equal to 100 mPa•s, and the average diameter of bubbles included in the foam at 30 seconds after the foam is generated by mixing the cleansing composition with air being more than or equal to 10µm and less than or equal to 100µm.

According to another embodiment, there is provided a method of generating foam by mixing the cleansing composition with air, including supplying the cleansing composition to a screw rotated by a motor; and mixing the cleansing composition with air by rotating the screw.

According to another embodiment, there is provided foam generated by mixing the cleansing composition with air.

According to another embodiment, there is provided a method of cleansing hair by using the foam.

### BRIEF DESCRIPTION OF DRAWINGS

Fig. 1 is a cross-sectional view of an example of a foam generating apparatus including a screw;
Fig. 2 is a diagram showing the viscosity of a foam cleansing agent at 10 to 40 seconds after foam is generated;
Fig. 3 is a diagram showing average diameters of bubbles of the foam cleansing agent at 30 and 60 seconds after foam is generated;
Fig. 4 is an outer perspective view of a foam generating apparatus that generates foam for cleansing of an embodiment;
Fig. 5 is a diagram showing the inner structure of the foam generating apparatus;
Fig. 6 is a diagram showing an example where the foam generating apparatus is placed in a beauty salon;
Fig. 7 is a diagram showing the foam viscosity of foam for cleansing of an embodiment in comparison with the foam viscosity of foam for cleansing generated by a conventional method of generating foam;
Fig. 8 is a diagram showing compositions of cleansing compositions used for generating foam for cleansing of an embodiment;
Fig. 9 is a diagram showing bubbles included in foam for cleansing generated by a method of generating foam of an embodiment and the foam generating apparatus in comparison with foam for cleansing generated by a conventional method of generating foam;
Fig. 10 is a diagram showing sizes of bubbles included in foam for cleansing generated by a method of generating foam of an embodiment and the foam generating apparatus in comparison with sizes of bubbles included in foam for cleansing generated by a conventional method of generating foam; and
Fig. 11 is a diagram for explaining an example of a method of cleansing hair using foam for cleansing of an embodiment.

### DETAILED DESCRIPTION OF THE PREFERRED EMBODIMENTS

Next, embodiments of the present invention will be described below with reference to drawings.

A composition for foam cleansing agent of the embodiment includes water and a surfactant and is used by generating foam so that the foam viscosity of the foam cleansing agent at 30°C at 10 seconds after the foam is generated by mixing the foam cleansing agent with air, and the average diameter of bubbles in the foam cleansing agent at 30 seconds after the foam is generated by mixing the foam cleansing agent with air, are 40 to 100 mPa•s and 10 to 100µm, and more preferably, 50 to 70 mPa•s and 10 to 80µm, respectively. At this time, the content of the surfactant in the composition for foam cleansing agent is 0.4 to 12% by weight, and more preferably 5 to 10% by weight. The viscosity of the composition for foam cleansing agent at 30°C may be 5 to 1500 mPa•s, and may be preferably less than or equal to 300 mPa•s.

When the foam viscosity at 30°C at 10 seconds after the foam is generated exceeds 100 mPa•s, or when the average diameter of bubbles at 30 seconds after the foam is generated is less than 10µm, the foam becomes in a glossy state or a liquid state so that the foam consistency may not be maintained. Therefore, a fine and creamy foam cleansing agent cannot be obtained to lower detergency and sensation. On the other hand, when the foam viscosity at 30°C at 10 seconds after the foam is generated is less than 40 mPa•s, or when the average diameter of bubbles at 30 seconds after the foam is generated is more than 100µm, a fine and creamy condition may not be maintained.

When the content of the surfactant in the composition for foam cleansing agent is less than 0.4% by weight, a fine and creamy foam cleansing agent may not be obtained to lower detergency and sensation, while when the content of the surfactant in the composition for foam cleansing agent is more than 12% by weight, a discharge amount of the surfactant when cleansing may be increased.

When the viscosity of the composition for foam cleansing agent at 30 °C is less than 5 mPa•s or more than 1500 mPa•s, a fine and creamy foam cleansing agent may not be obtained to lower detergency and sensation.

For the composition for foam cleansing agent of the embodiment, the foam viscosity of the foam cleansing agent at 30°C at 40 seconds after the foam is generated by mixing air and the average diameter of bubbles of the foam cleansing agent at 60 seconds after the foam is generated by mixing air may be more than or equal to 35 mPa•s and less than or equal to 150µm, and more preferably, more than or equal to 40 mPa•s and less than or equal to 130µm, respectively.

When the foam viscosity of the foam cleansing agent at 30°C at 40 seconds after the foam is generated is less than 35 mPa•s, or when the average diameter of bubbles of the foam cleansing agent at 60 seconds after the foam is generated is more than 150µm, a fine and creamy condition may not be maintained.

Here, the viscosity of the composition for foam cleansing agent and the foam viscosity of the foam cleansing agent may be measured by a B-type viscometer and a tuning fork vibration viscometer SV-10 (manufactured by A&D Company, Limited), respectively.

Further, the average diameter of bubbles of the foam cleansing agent may be calculated from an image obtained by a digital microscope VHX-200 (manufactured by Keyence Corporation) after mounting the foam cleansing agent on a slide glass, and covering by a cover glass.

For the surfactant, not specifically limited,
a cationic surfactant such as a stearyltrimethylammonium chloride, a docosyltrimethylammonium chloride, a dicocoylethyl hydroxyethylmonium methosulfate or the like;
an anionic surfactant such as an acylmethyl taurine sodium salt, a sodium methyl cocoyl taurate, a sodium polyoxyethylene laurylether sulfate (sodium laureth sulfate) or the like; or
an amphoteric surfactant such as a cocamidopropyl betaine, a 2-alkyl-N-carboxymethyl-N-hydroxyethylimidazolinium betaine (sodium cocoamphoacetate/alcohol/water) or the like may be used and a combination of two or more of these may also be used. Among these, the anionic surfactant and the amphoteric surfactant may be preferably combined in the light of detergency.

When the amphoteric surfactant is used as the surfactant, the composition for foam cleansing agent of the embodiment may further include a cationic polymer or an anionic polymer.

For the cationic polymer, not specifically limited, a cationic cellulose, a cationic locust bean gum, a cationic starch, a propyltrimoniumchloride acrylamide/dimethylacrylamide copolymer or the like may be used, and a combination of two or more of these may also be used. Among these, a cationic cellulose and/or a propyltrimoniumchloride acrylamide/dimethylacrylamide copolymer may be preferably used as sensation of the foam cleansing agent becomes well.

The content of the cationic polymer in the composition for foam cleansing agent may be, generally, 0.1 to 1% by weight, and more preferably, 0.3 to 0.5% by weight. When the content of the cationic polymer is less than 0.1% by weight, the sensation of the foam cleansing agent may be lowered, while when the content of the cationic polymer is more than 1% by weight, the hair of a user may become hard by cleansing.

For the anionic polymer, not specifically limited, a carrageenan, a xanthan gum or the like may be used, and a combination of two or more of these may also be used. Among these, a xanthan gum may be preferably used as the sensation of the foam cleansing agent becomes well.

The content of the anionic polymer of the composition for foam cleansing agent may be, generally, 0.01 to 0.5% by weight, and more preferably, 0.05 to 0.1% by weight. When the content of the anionic polymer is less than 0.01% by weight, the sensation of the foam cleansing agent may be lowered, while when the content of the anionic polymer is more than 0.5% by weight, the hair of a user may become hard by cleansing.

The composition for foam cleansing agent of the embodiment may further include a moisturizing agent, fragrance, an antiseptic agent, a pH stabilizer, a hair remedy ingredient or the like.

Further, the foam cleansing agent may be used for cleansing hair, skin or the like, and may be used as a shampoo, a body shampoo, a face-wash or the like. Among these, the foam cleansing agent is effective for a shampoo.

For the method of generating foam by mixing air into the composition for foam cleansing agent, not specifically limited provided that the foam viscosity of the foam cleansing agent at 30°C at 10 seconds after the foam is generated and the average diameter of bubbles of the foam cleansing agent at 30 seconds after the foam is generated becomes 40 to 100 mPa•s and between 10 to 100µm, respectively. However, a foam generating apparatus including a screw may be preferably used.

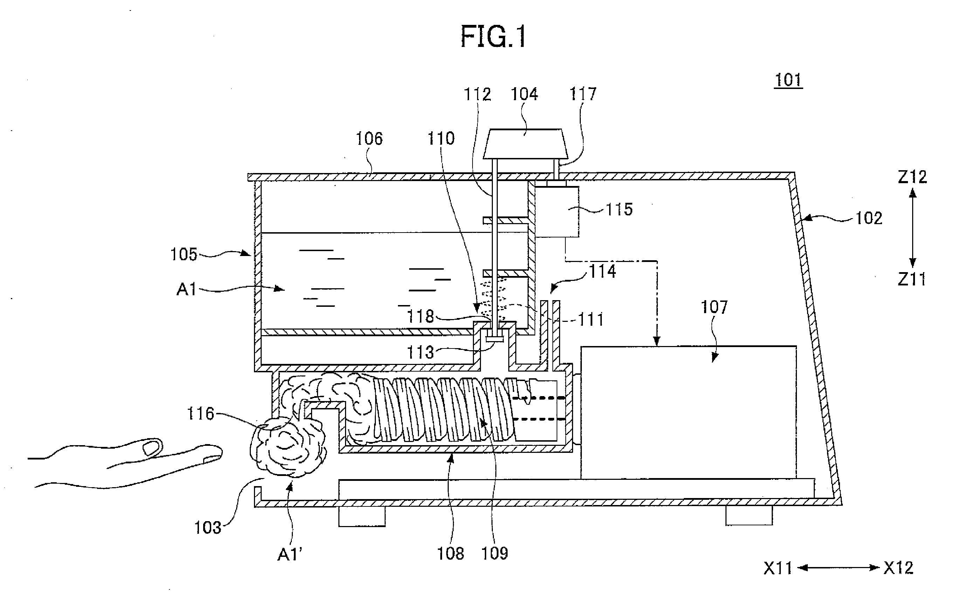

Fig. 1 shows an example of a foam generating apparatus including a screw. A foam generating apparatus 101 is provided with a hand inserting portion 103 at a front surface side of a body portion 102. Further, an operation portion 104 is provided on the body portion 102. When a user of the foam generating apparatus 101 operates the operation portion 104, as will be described later, a foam cleansing agent A1' is discharged from a discharge port 116. Further, a cover member 106 is provided at the top of the body portion 102. The cover member 106 is opened when a composition for foam cleansing agent A1 is fed into a liquid tank 105 in the body portion 102.

The tank 105, a motor 107, a foam generation room 108, a screw 109 and an open valve 110 are provided in the foam generating apparatus 101.

The operation portion 104 is provided with two shafts 112 and 117. The shaft 112 composes a part of the open valve 110, which will be explained later, and is movably attached to the body portion 102 in an upper and lower direction (Z11-Z12 direction).

The tank 105 is filled with the composition for foam cleansing agent A1. When the amount of the composition for foam cleansing agent A1 in the tank 105 decreases, a user opens the cover member 106 provided at the body portion 102, and supplies the composition for foam cleansing agent A1 into the tank 105. Thus, the foam generating apparatus 101 is configured such that the composition for foam cleansing agent A1 is capable of being supplied.

The motor 107 is configured to be driven and terminated by an ON/OFF operation of a switch 115. The switch 115 is connected to the shaft 117 which is provided to the operation portion 104, and the ON/OFF operation of the switch 115 is performed by a pushing operation of the operation portion 104 (operation toward a Z11 direction).

A rotating shaft of the motor 107 protrudes inside the foam generation room 108. Although the rotating shaft of the motor 107 penetrates a wall portion of the foam generation room 108 to protrude inside the foam generation room 108, the penetrated portion is sealed to be fluid tight. Further, the screw 109 is provided to a protruded portion of the rotating shaft of the motor 107. Thus, the screw 109 is rotated by the motor 107.

The foam generation room 108 is in communication with the tank 105, and the open valve 110 is provided at a communicating portion 118 of the tank 105 and the foam generation room 108. A valve body 113 of the open valve 110 is configured to open and close the communicating portion 118 of the tank 105 and the foam generation room 108. Then, when the valve body 113 is opened, the composition for foam cleansing agent A1 is flowed into the foam generation room 108 from the tank 105. When the valve body 113 is closed, the flow of the composition for foam cleansing agent A1 from the tank 105 to the foam generation room 108 is terminated.

The valve body 113 is provided at a lower end portion of the shaft 112. Further, the shaft 112 is provided with a coil spring 111. The coil spring 111 continuously pushes the shaft 112 toward a direction (Z12 direction) in which the valve body 113 closes the communicating portion 118.

Further, an admission port 114 is provided above an end of the foam generation room 108 in an X12 direction. When the screw 109 is rotated, air is introduced from the admission port 114 to the foam generation room 108.

At the foam generating apparatus 101, when the operation portion 104 is operated to be pushed, the switch 115 is switched on via the shaft 117, and the motor 107 is driven. Further, as the motor 107 is driven, the screw 109 is started to be rotated in the foam generation room 108.

Further, when the operation portion 104 is operated to be pushed, the shaft 112 moves in the Z11 direction against a spring force of the coil spring 111 so that the valve body 113 is opened. With this, the composition for foam cleansing agent A1 in the tank 105 is flowed into the foam generation room 108 via the communicating portion 118.

The screw 109 generates foam in the composition for foam cleansing agent A1 as well as sending the foam out in an X11 direction by mixing and agitating the composition for foam cleansing agent A1 flowed into the foam generation room 108 with air. The discharge port 116 which is opened within the hand inserting portion 103 is provided at an end of the foam generation room 108 in the X11 direction and the foam cleansing agent A1' is discharged from the discharge port 116.

On the other hand, when the user releases the operation to push the operation portion 104, the shaft 112 is moved in the Z12 direction by a resilient restoring force of the coil spring 111 to close the communicating portion 118 by the valve body 113. With this, the flow of the composition for foam cleansing agent A1 from the tank 105 to the foam generation room 108 is terminated. Further, with the movement of the shaft 112 in the Z12 direction, the operation portion 104 moves in the Z12 direction and the shaft 117 also moves in the Z12 direction. With this, the switch 115 is switched off so that the motor 107 is terminated. Further, by switching off of the switch 115, the rotation of the screw 109 is also terminated to terminate discharging of the foam cleansing agent A1'.

A method of cleansing hair, skin or the like by the foam cleansing agent A1' is not specifically limited, and a method of cleansing hair, skin or the like by a known cleansing agent may be used. Further, although the foam cleansing agent A1' at immediately after the foam is generated by mixing air may be preferably used for cleansing hair skin or the like considering detergency and sensation, however, it is not limited provided that the foam cleansing agent A1' is fine and creamy. For example the foam cleansing agent A1' at 30 seconds after the foam is generated by mixing air may be used.

### [example 1-1]

cocamidopropyl betaine solution: 3.6% by weight
2-alkyl-N-carboxymethyl-N-hydroxyethyl imidazoliniumbetaine (sodium cocoamphoacetate/alcohol/water): 3.6% by weight
acylmethyl taurine sodium salt: 2.5% by weight
sodium polyoxyethylene laurylether sulfate: 0.6% by weight
cationic cellulose: 0.3% by weight
propyltrimoniumchloride acrylamide /dimethylacrylamide copolymer: 0.5% by weight
xanthan gum: 0.05% by weight
dicocoylethyl hydroxyethylmonium methosulfate: 0.3% by weight
sorbitol solution: 3.0% by weight
sodium benzoate: 0.3% by weight
anhydrous citric acid solution: dosage purified water: balance

The composition for foam cleansing agent A1 was prepared by mixing the above ingredients by a usual method. The viscosity of the composition for foam cleansing agent A1 measured by a B type viscometer at 30°C was 45 mPa•s.

The foam cleansing agent A1' is generated by generating foam or bubbles in the composition for foam cleansing agent A1 by a lathering apparatus (lathermate, manufactured by Okuno denkisangyosha) having the same structure as the foam generating apparatus including the screw shown in Fig. 1.

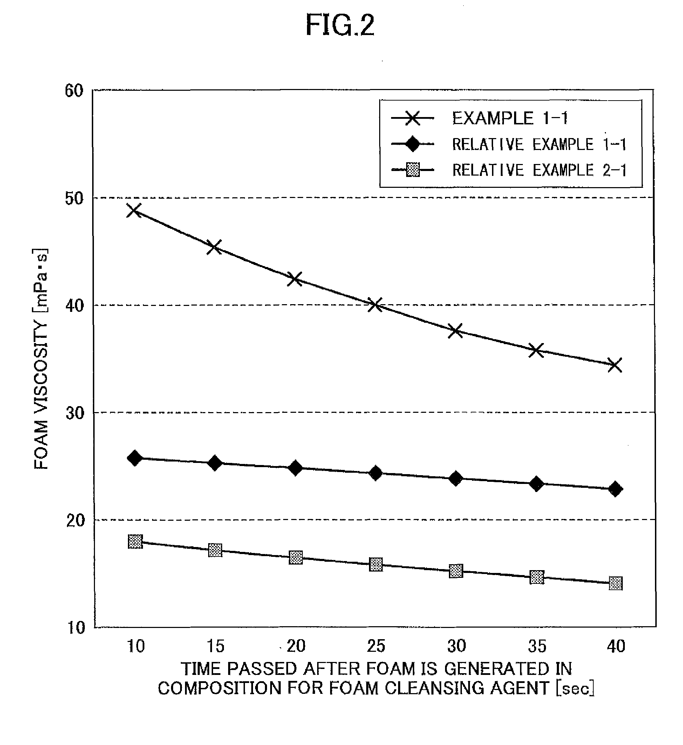

Fig. 2 shows results of measurements of the foam viscosities of the foam cleansing agent A1' at 10, 20, 30 and 40 seconds after the foam is generated. Here, the foam viscosity of the foam cleansing agent A1' is an average value of results obtained by measurements at 30°C of 7 times using a tuning fork vibration viscometer SV-10 (manufactured by A&D Company, Limited).

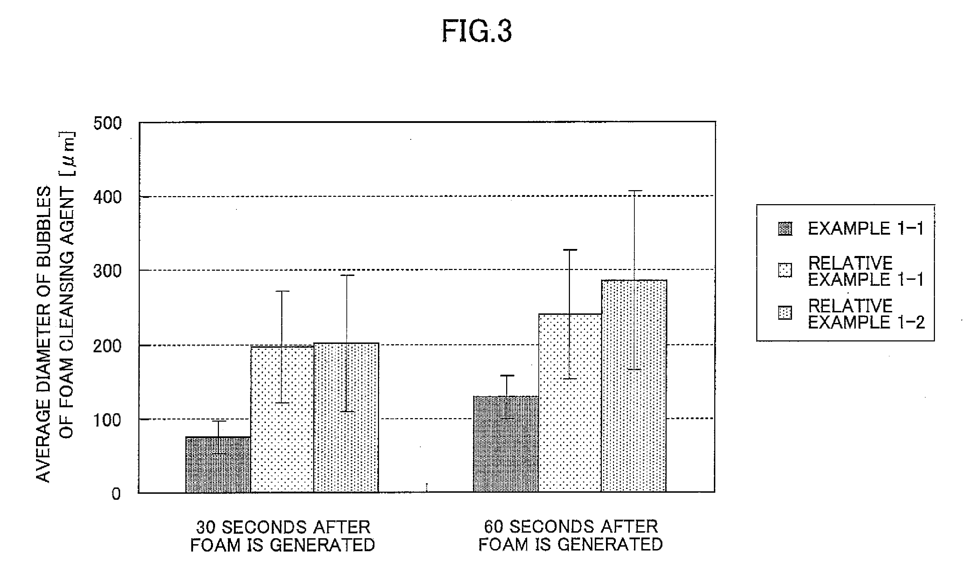

Fig. 3 shows average diameters of bubbles of the foam cleansing agent A1' at 30 and 60 seconds after the foam is generated. Here, the average diameter of bubbles of the foam cleansing agent A1' was calculated from an image obtained by a digital microscope VHX-200 (manufactured by Keyence Corporation) after mounting the foam cleansing agent A1' on a slide glass, and covering by a cover glass.

Then, hair was cleansed by the foam cleansing agent A1' at immediately after the foam is generated to reveal that a fine and creamy condition was maintained and the detergency and sensation of the foam cleansing agent A1' was well.

As described above, it can be understood that the foam cleansing agent A1' whose foam viscosity and average diameter of bubbles at 10 seconds after the foam is generated are 40 to 100 mPa•s and 10 to 100µm, respectively, is superior in detergency and sensation even when the content of the surfactant is small.

### [relative example 1-1]

A foam cleansing agent A1' was generated by a method similar to the example 1-1 except that a commercially available pump foamer container (manufactured by Yoshinokogyosho) adopting two meshes was used for generating foam or bubbles in the composition for foam cleansing agent A1, instead of the lathering apparatus (manufactured by Okuno denkisangyosha). Then, the foam viscosities of the foam cleansing agent A1' at 10, 20, 30 and 40 seconds after the foam is generated were measured.

Fig. 2 shows results of measurements of foam viscosities of the foam cleansing agent A1' at 10, 20, 30 and 40 seconds after the foam is generated.

Fig. 3 shows average diameters of bubbles of the foam cleansing agent A1' at 30 and 60 seconds after the foam is generated.

Then, hair was cleansed by the foam cleansing agent A1' at immediately after the foam is generated to reveal that the foam easily disappears, a fine and creamy condition was not maintained, and the detergency and sensation of the foam cleansing agent A1' was low.

### [relative example 1-2]

A foam cleansing agent A1' was generated by the similar method as the example 1-1 except that a set for cleansing face ("Awa-wash sengan set") EH2611P (Manufactured by Panasonic Corporation) was used for generating foam or bubbles in the composition for foam cleansing agent A1, instead of the lathering apparatus (manufactured by Okuno denkisangyosha). Then, the foam viscosities of the foam cleansing agent A1' at 10, 20, 30 and 40 seconds after the foam is generated were measured.

Fig. 2 shows results of measurements of foam viscosities of the foam cleansing agent A1' at 10, 20, 30 and 40 seconds after the foam is generated.

Fig. 3 shows average diameters of bubbles of the foam cleansing agent A1' at 30 and 60 seconds after the foam is generated.

Then, hair was cleansed by the foam cleansing agent A1' at immediately after the foam is generated to reveal that the foam easily disappears, a fine and creamy condition was not maintained, and the detergency and sensation of the foam cleansing agent A1' was low.

As a result, for the composition for foam cleansing agent A1, as the content of the surfactant is small, even when foam is generated in the composition for foam cleansing agent A1 by using the pump foamer container (manufactured by Yoshinokogyosho) or the set for cleansing face ("Awa-wash sengan set") EH2611P (Manufactured by Panasonic Corporation), the foam cleansing agent A1' with low detergency and sensation is generated. On the other hand, when foam is generated in the composition for foam cleansing agent A1 such that the foam viscosity of the foam cleansing agent A1' at 10 seconds after the foam is generated and the average diameter of bubbles of the foam cleansing agent A1' at 30 seconds after the foam is generated becomes 40 to 100 mPa•s and 10 to 100µm, respectively, the foam cleansing agent A1' with superior detergency and sensation can be obtained.

Other examples of the composition for foam cleansing agent A1 are shown in the following.

### [composition for foam cleansing agent (for hair shampoo)]

The composition for foam cleansing agent (for hair shampoo) is prepared by mixing 4.0% by weight of a lauryldimethylamino acetic acid betaine and 0.2% by weight of a stearyltrimethylammonium chloride as the surfactant, a propylene glycol, a cationic cellulose, a xanthan gum, a sodium benzoate, a citric acid, a 2-phenoxyethanol, a colorant, fragrance, and purified water by a usual method so that its viscosity at 30°C becomes 5 to 1500 mPa•s.

### [composition for foam cleansing agent (for hair shampoo)]

The composition for foam cleansing agent (for hair shampoo) is prepared by mixing 0.5% by weight of a sodium polyoxyethylene laurylether sulfate, 0.5% by weight of a sodium methyl cocoyl taurate and 8.0% by weight of cocamidopropyl betaine solution as the surfactant, a cationic guar gum, a stearoxy hydroxylpropylamine, sorbitol solution, an L-arginine, a hydroxyethylurea, a colorant, fragrance, and purified water by a usual method.

### [composition for foam cleansing agent (for hair shampoo)]

The composition for foam cleansing agent (for hair shampoo) is prepared by mixing 6.0% by weight of a disodium laureth sulfosuccinate, 2.0% by weight of a 2-alkyl-N-carboxymethyl-N-hydroxyethylimidazolinium betaine and 1.0% by weight of cocamidopropyl betaine solution as the surfactant, a polyquaternium-6, a glycerin, a cationic locust bean gum, a trimethyldodecylammonium chloride, a colorant, fragrance, and purified water by a usual method.

### [composition for foam cleansing agent (for hair shampoo)]

The composition for foam cleansing agent (for hair shampoo) is prepared by mixing 2.0% by weight of a lauric acid taurine sodium salt, 3.0% by weight of a sodium polyoxyethylene laurylether sulfate, 1.0% by weight of a lauryldimethylamino acetic acid betaine and 2.0% by weight of cocamidopropyl betaine solution as the surfactant, a propyltrimoniumchloride acrylamide/dimethylacrylamide copolymer (mixture of purified water), sorbitol solution, a succinic acid, camellia oil, a colorant, fragrance and purified water by a usual method.

### [composition for foam cleansing agent (for body shampoo)]

The composition for foam cleansing agent (for body shampoo) is prepared by mixing 6.0% by weight of a lauric acid triethanolamine and 2.0% by weight of a lauryldimethylamino acetic acid betaine as the surfactant, a glycerin, a dipropylene glycol, chamomilla extract, an edetate trisodium, an antiseptic agent, a colorant, fragrance and purified water by a usual method.

### [composition for foam cleansing agent (for cleansing foam for face)]

The composition for foam cleansing agent (for cleansing foam for face) is prepared by mixing 0.05% by weight of a stearic acid, 2.0% by weight of a lauric acid, 3.0% by weight of a myristic acid, 1.8% by weight of a potassium hydroxide, 0.3% by weight of a coconut diethanolamide and 2.0% by weight of cocamidopropyl betaine solution as the surfactant, a glycerin, a polyethylene glycol 1500, sorbitol solution, melissa extract, an edetate trisodium, fragrance and purified by a usual method.

### [composition for foam cleansing agent (for cleansing foam for face)]

The composition for foam cleansing agent (for cleansing foam for face) is prepared by mixing 2.0% by weight of an N-methyltaurine sodium salt and 1.0% by weight of an lauryldimethylamino acetic acid betaine as the surfactant, a glycerin, a dipropylene glycol, an 1,3-butylene glycol, a polyethylene glycol 1500, sorbitol solution, a lauric acid, a myristic acid, rosa roxburghii fruit extract, a dipotassium glycyrrhizate, an edetate trisodium, fragrance and purified water by a usual method.

### [composition for foam cleansing agent (for hand soap)]

The composition for foam cleansing agent (for hand soap) is prepared by mixing 3.0% by weight of a lauric acid, 1.0% by weight of a myristic acid, 2.5% by weight of a triethanolamine, 1.0% by weight of a disodium laureth sulfosuccinate, 0.5% by weight of a coconut diethanolamide and 2.0% by weight of cocamidopropyl betaine solution as the surfactant, a propylene glycol, a sodium chloride, an edetate trisodium, fragrance and purified water by a usual method.

### [composition for foam cleansing agent (for hand soap)]

The composition for foam cleansing agent (for hand soap) is prepared by mixing 6.0% by weight of sodium C14-16 olefin sulfonate solution as the surfactant, a propylene glycol, a polyoxyethylene lauryl ether, a malic acid, eucalyptus oil, a sodium benzoate, benzalkonium chloride solution, an edetate trisodium and purified water by a usual method.

Another embodiment will be explained with reference to drawings.

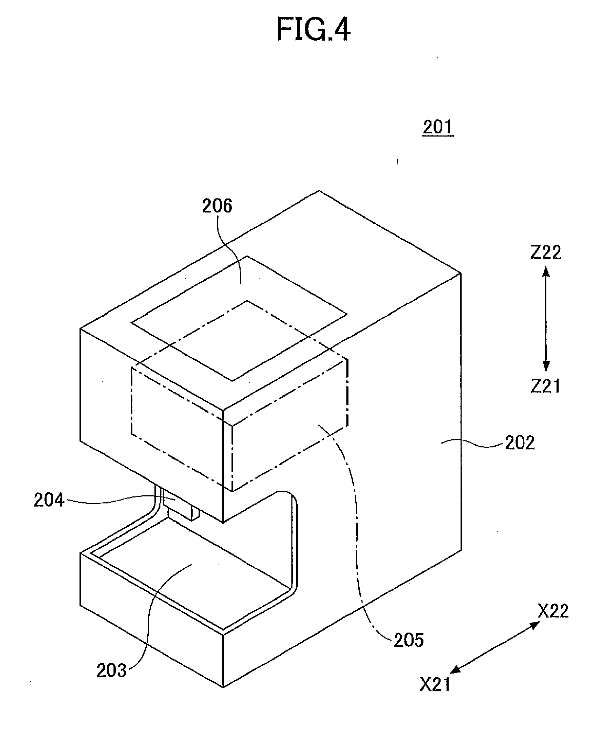

Fig. 4 shows a foam generating apparatus 201 used for generating foam A2 for cleansing of an embodiment. Before explaining a characteristic of the foam A2 for cleansing, a method of foaming the foam A2 for cleansing of an embodiment, and the foam generating apparatus 201 used for performing the method will be explained.

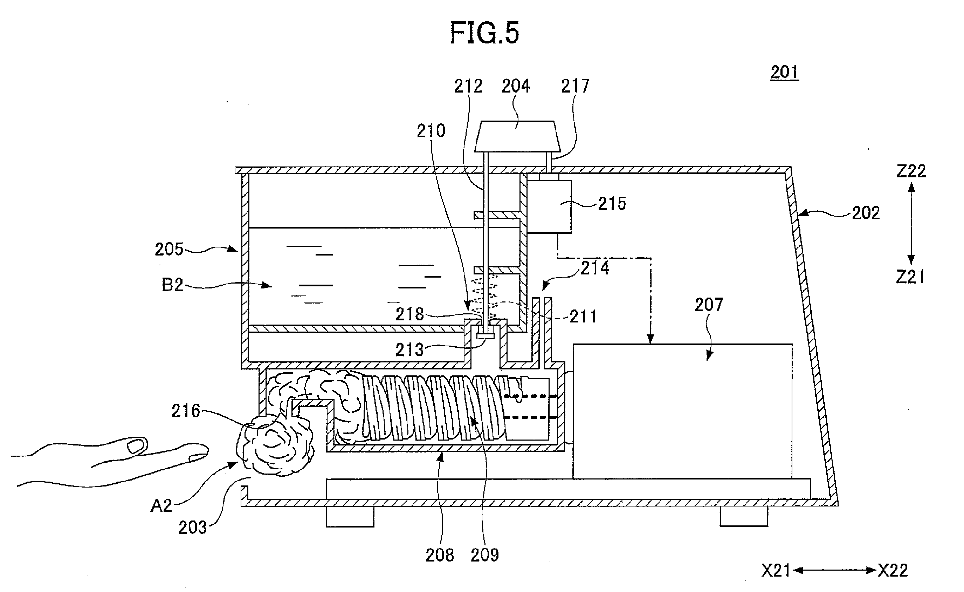

Fig. 4 is an outer perspective view of the foam generating apparatus 201, and Fig. 5 is a cross-sectional view showing the structure of the foam generating apparatus 201. In this embodiment, the foam generating apparatus 201 which uses a shampoo as original liquid of cleansing composition B2 is described as an example. However, the original liquid of cleansing composition B2 is not limited to a shampoo, and the foam generating apparatus 201 is applicable to various original liquid of cleansing composition such as a hand soap, a body shampoo or the like which are preferably used in a foam form.

As shown in Fig. 4, the foam generating apparatus 201 is provided with a hand inserting portion 203 at a front surface side of the body portion 202. An operation portion 204 is provided within the hand inserting portion 203. The foam generating apparatus 201 is configured, as will be explained later, such that the foam A2 for cleansing is discharged out from a foam discharge port 216 when a user of the foam generating apparatus 201 operates the operation portion 204 while inserting one's hand in the hand inserting portion 203. Further, a cover member 206 is provided at the top of the body portion 202. The cover member 206 is opened when the original liquid of cleansing composition B2 is fed into a liquid tank 205 in the body portion 202.

Next, the inner structure of the foam generating apparatus 201 is explained with reference to Fig. 5. The foam generating apparatus 201 includes inside, the liquid tank 205, a motor 207, a foam generation room 208, a screw 209, an open valve 210 or the like. As described above with reference to Fig. 4, the operation portion 204 is provided within the hand inserting portion 203, however, it is described in Fig. 5 such that the operation portion 204 is provided at an upper portion of the body portion 202 for explanation.

As described above, the operation portion 204 is operated by the user of the foam generating apparatus 201. The operation portion 204 is provided with two shafts 212 and 217. The shaft 212 composes a part of the open valve 210, which will be explained later, and is movably attached to the body portion 202 in an upper and lower direction (Z21-Z22 direction).

The liquid tank 205 is filled with original liquid of cleansing agent (original liquid of cleansing composition B2). When the amount of the original liquid of cleansing composition B2 in the liquid tank 205 decreases, the cover member 206 provided at the body portion 202 is opened and the original liquid of cleansing composition B2 is fed into the liquid tank 205 therefrom. As this, for the foam generating apparatus 201, as it is possible to supply the original liquid of cleansing composition B2, environmental measures can be improved and cost can be reduced compared with a case where a container such as a conventional aerosol container which is discarded every time.

The motor 207 is configured to be driven and terminated by an ON/OFF operation of a switch 215. The switch 215 is connected to the shaft 217 which is provided to the operation portion 204, and the ON/OFF operation of the switch 215 is performed by a pushing operation of the operation portion 204 (operation toward a Z21 direction).

A rotating shaft of the motor 207 protrudes inside the foam generation room 208. Although the rotating shaft of the motor 207 penetrates a wall portion of the foam generation room 208 to protrude inside the foam generation room 208, the penetrated portion is sealed to be fluid tight. Further, the screw 209 is provided to a protruded portion of the rotating shaft of the motor 207. Thus, the screw 209 is rotated by the motor 207.

The foam generation room 208 is in communication with the liquid tank 205. The open valve 210 is provided at a communicating portion 218 of the liquid tank 205 and the foam generation room 208. A valve body 213 of the open valve 210 is configured to open and close the communicating portion 218 of the liquid tank 205 and the foam generation room 208. Then, when the valve body 213 is opened, the original liquid of cleansing composition B2 is flowed into the foam generation room 208 from the liquid tank 205. When the valve body 213 is closed, the flow of the original liquid of cleansing composition B2 from the liquid tank 205 to the foam generation room 208 is terminated.

The valve body 213 is provided at a lower end portion of the above described shaft 212. Further, the shaft 212 is provided with a coil spring 211, and the coil spring 211 continuously pushes the shaft 212 toward a direction (Z22 direction) in which the valve body 213 closes the communicating portion 218.

Further, an air admission port 214 is provided above an end of the foam generation room 208 in an X22 direction shown by an arrow. When the screw 209 is rotated, air is introduced from the air admission port 214 to the foam generation room 208.

At thus structured foam generating apparatus 201, when the operation portion 204 is operated to be pushed, the switch 215 is switched on via the shaft 217, and the motor 207 is driven. Further, as the motor 207 is driven, the screw 209 is started to be rotated in the foam generation room 208.

Further, when the operation portion 204 is operated to be pushed, the shaft 212 moves in the Z21 direction against a spring force of the coil spring 211 so that the valve body 213 is opened. With this, the original liquid of cleansing composition B2 in the liquid tank 205 is flowed into the foam generation room 8 via the communicating portion 218.

The screw 209 has a function to generate the foam A2 for cleansing by mixing and agitating the original liquid of cleansing composition B2 which is flowed into the foam generation room 208 with air, and send it out in the X21 direction shown by an arrow. The foam discharge port 216 is provided at an end of the foam generation room 208 in the X21 direction shown by an arrow, which is opened within the hand inserting portion 203. The foam A2 for cleansing generated in the foam generation room 208 by the screw 209 is discharged from the foam discharge port 216.

On the other hand, when the user releases the operation to push the operation portion 204, the shaft 212 is moved upward (in the Z22 direction) by a resilient restoring force of the coil spring 211 to close the communicating portion 218 by the valve body 213. With this, the flow of the original liquid of cleansing composition B2 from the liquid tank 205 to the foam generation room 208 is terminated. Further, with the upward movement of the shaft 212, the operation portion 204 moves upward and the shaft 217 also moves upward. With this, the switch 215 is switched off so that the motor 207 is terminated. Further, by switching off of the switch 215, the rotation of the screw 209 is also terminated to terminate discharging of the foam A2 for cleansing.

Here, as shown in Fig. 4, for the actual foam generating apparatus 201, the operation portion 204 is provided within the hand inserting portion 203. Therefore, when the user inserts one's hand in the hand inserting portion 203 and operates the operation portion 204, the user can receive the foam A2 for cleansing discharged from the foam discharge port 216 on one's palm.

According to the above described method of generating the foam A2 for cleansing and the foam generating apparatus 201, the fine and creamy foam A2 for cleansing can be generated as the original liquid of cleansing composition B2 and air are mixed by the rotation of the screw 209 rotated by the motor 207 (which will be explained later).

Further, as a mesh member is not used, different from a method of generating foam using a conventional pump foamer, blocking does not occur to extend the lifetime. Further, although for the pump foamer, it is necessary to strongly push a button and the usability is bad, for the foam generating apparatus 201, the usability can be improved because the foam A2 for cleansing is discharged on one's palm just by inserting the hand in the hand inserting portion 203 and operating the operation portion 204.

Further, according to the method of generating foam of the embodiment, as flammable liquid gas (propellant) which is necessary for an aerosol container is not used, safety can be improved. Further, as the original liquid of cleansing composition B2 can be supplied to the liquid tank 205, it is not necessary to discard every time as the aerosol container, and further vaporization of liquid gas does not occur and an environmentaly-friendly method of generating foam can be actualized.

Further, for the method of generating foam of the embodiment and the foam generating apparatus 201, the foam A2 for cleansing is generated immediately after the motor 207 is driven and the screw 209 is rotated. Therefore, a time necessary for obtaining the foam A2 for cleansing is short to improve usability in this point as well.

Next, the characteristic of the foam A2 for cleansing generated as described above is explained.

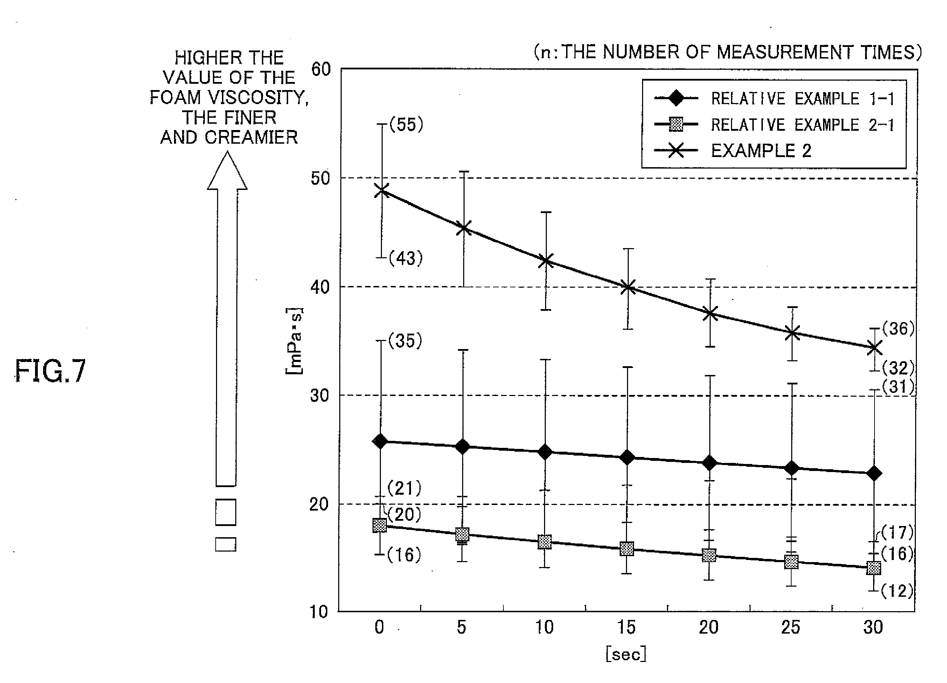

Fig. 7 is a diagram showing the foam viscosity of the foam A2 for cleansing generated by the above method of generating foam and the foam generating apparatus 201 in comparison with the foam viscosity of foam for cleansing generated by a conventional method of generating foam. The measurement of the foam viscosities were conducted by a tuning fork vibration viscometer (SV-10, manufactured by A&D Company, Limited).

A relative example 2-1 shown in the drawing is the foam viscosity of foam for cleansing generated by a conventional pump foamer. Further, a relative example 2-2 shown in the drawing is the foam viscosity of foam for cleansing generated by an electric method of generating foam using a conventional expanded stone. Further, an example 2 in the drawing is the foam viscosity of the foam A2 for cleansing generated by the method of generating foam and the foam generating apparatus 201 which is explained with reference to Fig. 4 and Fig. 5. Here, the numeral shown as (n= ) in the drawing express the number of measurement times, and the shown characteristic lines are average value of them.

Further in the drawing, the foam viscosity (mPa•s) is shown in the ordinate axis, and the time (seconds) after the foam for cleansing is generated is shown in the abscissa axis. Here, the fineness and creaminess of the foam correlates with the foam viscosity where higher the value of the foam viscosity, the finer and creamier the foam becomes. Further, for the time, it is assumed that the time when the foam for cleansing is generated is 10 seconds and subsequent measurements were conducted every 5 seconds until 40 seconds.

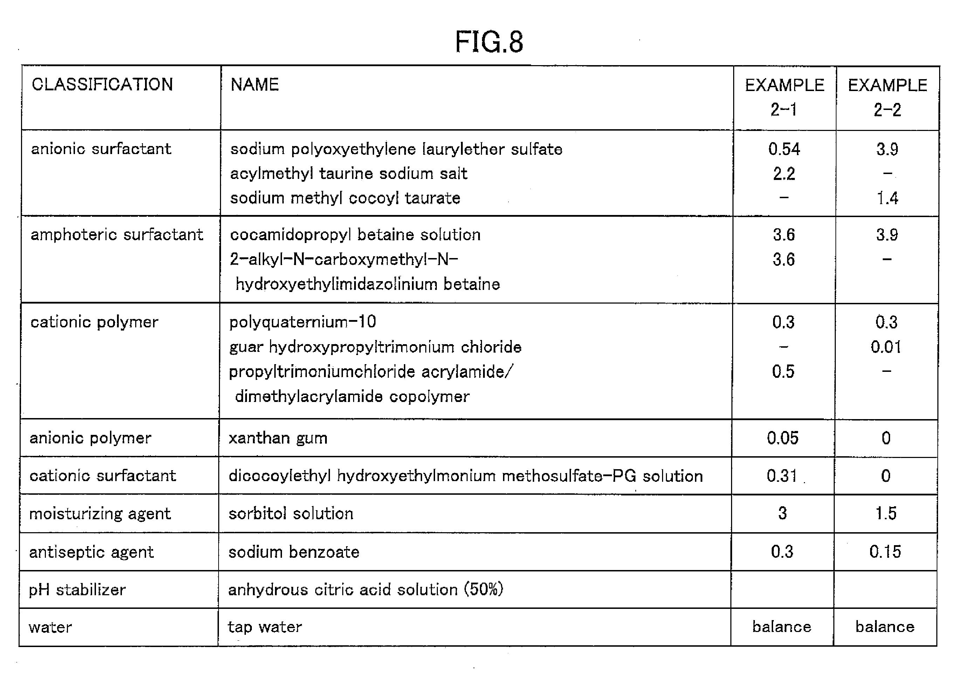

The original liquid of cleansing composition was the same for the relative examples 2-1 and 2-2, and the example 2. Specifically, the original liquid of cleansing composition having the composition shown as the example 2-1 in Fig. 8 was used. The original liquid of cleansing composition of the example 2-1 includes a surfactant (anionic surfactant and amphoteric surfactant), and the content of the surfactant is more than or equal to 0.4% by weight and less than or equal to 12% by weight. Thus, the content of the surfactant is set less than that of a general original liquid of cleansing composition used as a shampoo or the like. Further, the viscosity (liquid viscosity) of the original liquid of cleansing composition of the example 2-1 at 30°C is more than or equal to 5 mPa•s and less than or equal to 1500 mPa•s.

In Fig. 7, referring to the foam viscosity of the foam A2 for cleansing generated by the method of generating foam of the embodiment shown as the example 2, the foam viscosity at immediately after the foam is generated is more than or equal to 43 mPa•s and less than or equal to 55 mPa•s. It is higher than that of the relative example 2-1 where the foam viscosity is more than or equal to 20 mPa•s and less than or equal to 35 mPa•s, and that of the relative example 2-2 where the foam viscosity is more than or equal to 16 mPa•s and less than or equal to 21 mPa•s. Thus, it is revealed that the foam A2 for cleansing generated by the method of generating foam of the embodiment is finer and creamier than the foam for cleansing generated by the conventional methods of generating foam. Further, even though the content of the surfactant included in the original liquid of cleansing composition is small, the fine and creamy foam for cleansing is generated and maintained after time has passed.

Next, the variation of the foam viscosity with time is described.

For the foam A2 for cleansing generated by the method of generating foam of the embodiment, the difference (variation with time) between the foam viscosity at immediately (10sec) after the foam is generated and the foam viscosity at 40 seconds after (40sec) the foam is generated is more than or equal to 7 mPa•s and less than or equal to 23 mPa•s. On the other hand, for the relative example 2-1, the variation with time of the foam viscosity is more than or equal to 4 mPa•s and less than or equal to 19 mPa•s, and for the relative example 2-2, the variation with time of the foam viscosity is more than or equal to 4 mPa•s and less than or equal to 9 mPa•s.

As described above, according to the foam A2 for cleansing of the embodiment, the decreasing rate of the foam viscosity with time is higher compared with those of the relative examples 2-1 and 2-2. However, even after 30 seconds have passed, the foam viscosity of the foam A2 for cleansing of the embodiment is more than the maximum value of that of the relative example 2-1 (31 mPa•s) and the maximum value of that of the relative example 2-2 (17 mPa•s). Thus, it is revealed that the foam A2 for cleansing of the embodiment is better at persistency of the foam, in other words foam maintenance, compared with the relative examples 2-1 and 2-2. It means that by forming fine and creamy foam, the persistency of the foam can be improved.

For the experiment measuring the foam viscosity of the foam A2 for cleansing shown in Fig. 7, the original liquid of cleansing composition having the composition shown as the example 2-1 in Fig. 8 was used. The inventors of the invention have conducted the similar measurement of the foam viscosity using the original liquid of cleansing composition shown as the example 2-2 instead of the original liquid of cleansing composition B2 of the example 2-1. The original liquid of cleansing composition of the example 2-2 has the composition which is similar to the conventional generally used shampoo.

Here, for the generally used conventional shampoo, the viscosity (liquid viscosity) of the original liquid at 30°C is more than or equal to 1500 mPa•s and the content of the surfactant is more than or equal to 12% by weight. Thus, if it is directly used as it is, it cannot be foam of the embodiment generated by mixing air into a cleansing composition such that the foam viscosity at immediately after the foam is generated is more than or equal to 40 mPa•s and less than or equal to 100 mPa•s as well as the average diameter of bubbles included in the foam at immediately after the foam is generated is more than or equal to 10µm and less than or equal to 100µm. Therefore, a composition which is adjusted such that the content of the surfactant and the viscosity (liquid viscosity) of the original liquid are adapted for the embodiment was used for original liquid of cleansing composition (hereinafter, referred to as adjusted original liquid).

As a result, foam for cleansing having the foam viscosity characteristic same as the example 2 shown in Fig. 7 was generated. It means that, by using the method of generating foam of the embodiment and foam generating apparatus 201, it is capable of using the adjusted original liquid as the original liquid of cleansing composition B2. For the case using the adjusted original liquid of the example 2-2, although the content of the surfactant may increase compared with a case using the original liquid of cleansing composition of the example 2-1, it is capable of actualizing sensation in using similar to a commercially available shampoo and cost can be reduced compared with the original liquid of cleansing composition of the example 2-1.

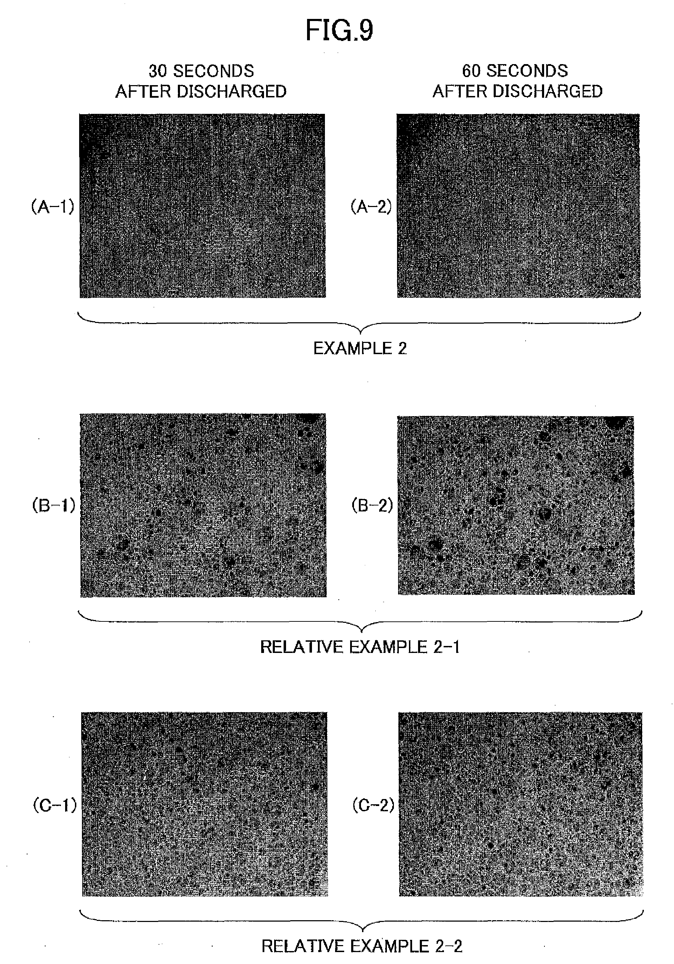

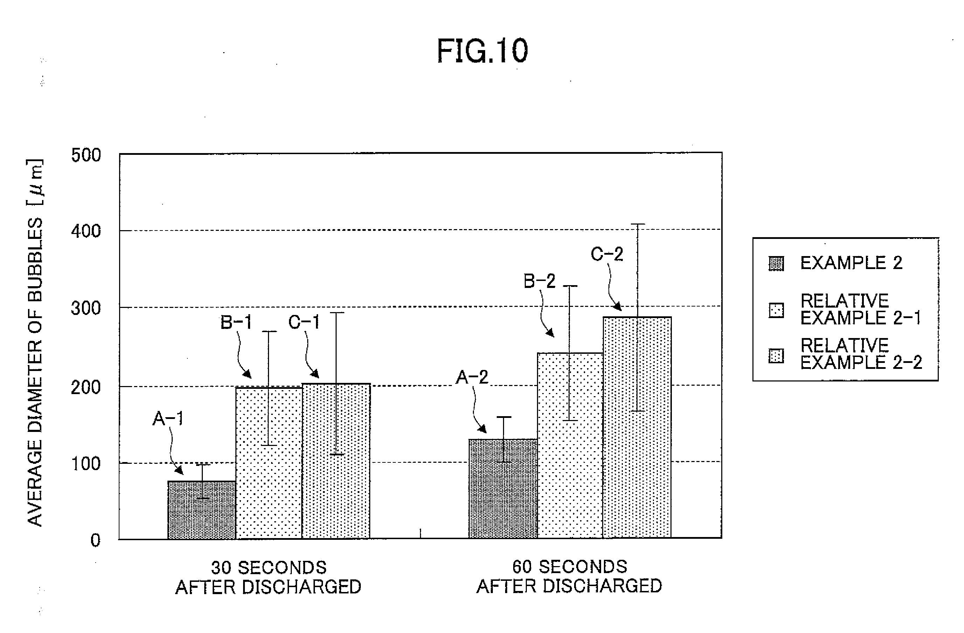

Fig. 9 shows bubbles included in the foam A2 for cleansing generated by the above method of generating foam and the foam generating apparatus 201, and bubbles generated by the foam for cleansing generated by the conventional methods of generating foam obtained by observing with a digital microscope. For the observation, a digital microscope (VHX-200, manufactured by Keyence Corporation) set at an enlarged scale of 50 times was used, and the bubbles generated by the respective methods of generating foam were observed at 30 or 60 seconds after discharged. For obtaining the images of the bubbles, the bubbles immediately after being generated were mounted on a slide glass and covered by a cover glass. Further, in Fig. 10, the sizes of the bubbles included in the foam A2 for cleansing generated by the above method of generating foam and the foam generating apparatus 201 and included in the foam for cleansing generated by the conventional methods of generating foam were compared by calculating the average diameters of the bubbles in the images obtained in Fig. 9.

In Fig. 9, (A-1) and (A-2) shown as the relative example 2-1 are images of the bubbles included in the foam for cleansing generated by the conventional pump foamer. Further, in Fig. 9, (B-1) and (B-2) shown as the relative example 2-2 are images of the bubbles included in the foam for cleansing generated by the electric method of generating foam using a conventional expanded stone. Further, in Fig. 9, (A-1) and (A-2) shown as the example 2 are images of the bubbles included in the foam A2 for cleansing generated by the method of generating foam and the foam generating apparatus 201 explained with reference to Fig. 4 and Fig. 5. Further, in Fig. 9, (A-1), (B-1) and (C-1) are images showing conditions at 30 seconds after being discharged, and (A-2), (B-2) and (C-2) are images showing conditions at 60 seconds after being discharged.

The used original liquid of cleansing composition was the same for the relative examples 2-1 and 2-2, and the example 2. Specifically, the original liquid of cleansing composition having the composition shown as the example 2-1 in Fig. 8 was used. The original liquid of cleansing composition of the example 2-1 includes a surfactant (anionic surfactant and amphoteric surfactant), and the content of the surfactant is more than or equal to 0.4% by weight and less than or equal to 12% by weight. Thus, the content of the surfactant is set less than that of a general original liquid of cleansing composition used as a shampoo or the like. Further, the viscosity. (liquid viscosity) of the original liquid of cleansing composition of the example 2-1 at 30°C is more than or equal to 5 mPa•s and less than or equal to 1500 mPa•s.

Further in Fig. 10, average values of the bubbles confirmed in three images for each of the cases are shown where the numeral shown as (n= ) expresses the number of measured bubbles. In Fig. 10, the diameter (µm) of the bubbles is shown in the ordinate axis where the average values of the bubbles at 30 and 60 seconds after the foam were generated in the example 2-1, and the relative examples 2-1 and 2-2 are shown.

It can be understood that the diameter of the bubbles included in the foam A2 for cleansing generated by the method of generating foam of the embodiment shown as the example 2 in Fig. 9 and Fig. 10 is extremely fine compared with those of the relative examples. Further, the average of the diameters of the bubbles is 80µm, which is extremely lower than that of the relative example 2-1 and the relative example 2-2 where the averages of the diameters of the bubbles are 200µm. Thus, the foam A2 for cleansing generated by the method of generating foam of the embodiment is revealed to be finer and creamier than the foam for cleansing generated by the conventional method of generating foam. Further, it is revealed that the finer and creamier foam for cleansing can be generated because the included bubbles are fine although the content of the surfactant included in the original liquid of cleansing composition is small.

Next, the variation of the foam viscosity with time is described.

For the foam A2 for cleansing generated by the method of generating foam of the embodiment (example 2), the average value of the diameters of the bubbles at 60 seconds after the foam is generated is 130µm, which is obviously lower than the average value of the diameters of the bubbles 240µm of the relative example 2-1 or 290µm of the relative example 2-2. This means that the fine and creamy foam is maintained longer compared with the foam for cleansing generated by the conventional methods of generating foam.

Next, a method of cleansing hair using the foam A2 for cleansing having the above described characteristic of the foam viscosity is explained.

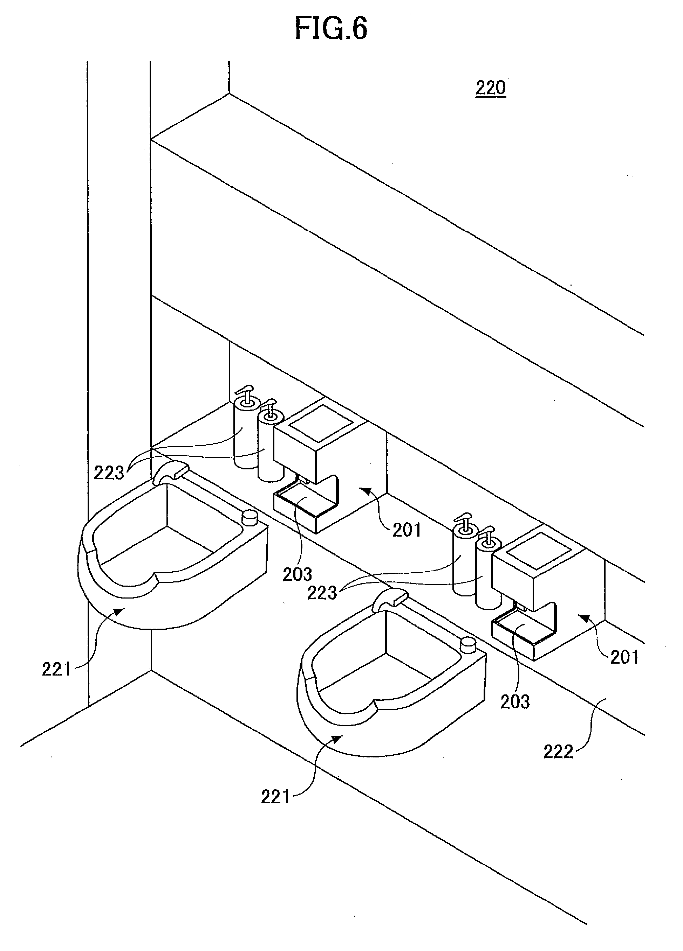

Fig. 6 shows a vicinity of a hair cleansing counter 221 (hereinafter, referred to as "shampoo counter") at a beauty salon 220. The above described foam generating apparatus 201, capable of generating the foam A2 for cleansing whose foam viscosity at immediately after the foam is generated is more than or equal to 40 mPa•s and the average value of the diameters of the bubbles at immediately after the foam is generated is less than or equal to 1mm, is placed at a shelf 222 of a beauty salon 220. The shelf 222 is where containers for cosmetics 223, towels used when cleansing hair or the like are placed. The foam generating apparatus 201 is placed at the shelf 222, which is the position where a staff of the salon who cleanses the hair of the client can easily access.

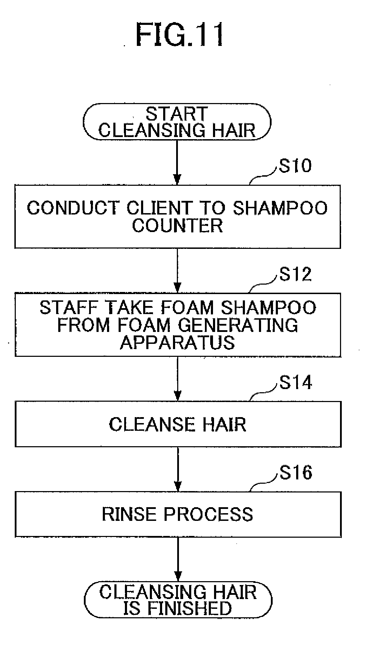

Fig. 11 is a flowchart showing a process for cleansing hair of the client by the staff of the salon. When cleansing of the hair is started, the staff of the salon conducts the client to the shampoo counter 221 (step S10). Then, after positioning the head of the client at the shampoo counter 221, the staff of the salon inserts one's hand in the hand inserting portion 203 of the foam generating apparatus 201 and operates the operation portion 204.

As described above, the foam generating apparatus 201 is configured that the fine and creamy foam A2 for cleansing is discharged from the foam discharge port 216 on one's palm immediately after the operation portion 204 is operated. Thus, the staff of the salon can easily take the foam A2 for cleansing having a good characteristic on one's hand without performing a troublesome pushing operation of a button of the pump foamer or the aerosol container (step S12).

Subsequently, the process for cleansing hair is started by putting the foam A2 for cleansing generated at the foam generating apparatus 201 on the hair of the client (step S14). According to the method of cleansing hair of the embodiment, as the foam A2 for cleansing in a foam form is discharged from the foam generating apparatus 201, it is unnecessary to perform lathering which is necessary in a conventional method when using a liquid shampoo. Thus, the process of cleansing hair for the staff of the salon can be eased.

Especially, for a woman having long hair, the staff of the salon generally generates foam near the scalp and moves the foam to the end of the hair to complete cleansing of the hair except the scalp. By the method of the embodiment, it is possible to directly put the foam taken on one's hand in step S12 to the end of the hair such that the end of the hair is smoothly cleansed in addition to the unnecessary step of lathering. As a result, the operability of the staff of the salon can be improved.

Further, as the foam A2 for cleansing has good foam maintenance, the foam of the foam A2 for cleansing can be maintained during cleansing the hair. With this, the sensation of fine and creamy foam can be applied to the client while cleansing the hair to give a good impression to the client.

After cleansing of hair by the foam A2 for cleansing is finished, the staff of the salon rinses the foam A2 for cleansing (step S16), and the process of cleansing of hair is finished.

As described above, as the content of the surfactant in the original liquid of cleansing composition B2, which is the original liquid for generating the foam A2 for cleansing, is less than that of a general shampoo, and by using the method of cleansing hair of the embodiment, it is unnecessary for the person to cleanse hair to perform a lathering process, the time necessary for touching the surfactant or water can be shortened so that a stimulation to skin or a trouble to the hand can be suppressed.

The inventors had a beauty salon perform the above described method of cleansing hair for two weeks and have 11 staffs of the salon who have performed the method of cleansing hair answer a questionnaire about sensation in using. As a result, by using the method of cleansing hair of the embodiment, a result that a stimulation to skin or trouble to the hand can be prevented compared with a conventional general method of cleansing hair was obtained.

Further, it is revealed that the amount of the foam A2 for cleansing can be reduced compared with a conventional liquid shampoo. Further, it is revealed that the foam A2 for cleansing was good in spreading onto the hair and can prevent twisting of the hair. As described, by using the method of cleansing hair of the embodiment, various merits which cannot be obtained by the conventional method of cleansing hair can be obtained.

Other examples of the cleansing composition for generating the foam for cleansing of the embodiment will be described. Here, these prescription examples are prepared by a usual method, but the fine and creamy foam for cleansing of a good persistency of the foam can be generated by generating foam by the foam generating apparatus 201, although the content of the surfactant of which is less than that of a generally used cleansing composition.

### [prescription example 2-3: composition for hair shampoo]

poly (oxyethylene) lauryl sulfonate triethanolamine salt: 5.0% by weight
lauryldimethylamino acetic acid betaine: 2.5% by weight
propylene glycol: 2.0% by weight
cationic cellulose: 0.3% by weight
sodium benzoate: 0.5% by weight
citric acid: 0.05% by weight
2-phenoxyethanol: 0.1% by weight
colorant: dosage
fragrance: dosage
purified water: balance

### [prescription example 2-4: composition for hair shampoo]

sodium polyoxyethylene laurylether sulfate: 1.5% by weight
sodium methyl cocoyl taurate: 0.8% by weight
cocamidopropyl betaine solution: 3.5% by weight
cationic guar gum: 0.3% by weight
sorbitol solution: 2.0% by weight
L-arginine: 0.08% by weight
hydroxyethylurea: 0.2% by weight
seaweed extract: dosage
colorant: dosage
fragrance: dosage
purified water: balance

### [prescription example 2-5: composition for hair shampoo]

disodium laureth sulfosuccinate: 6.0% by weight
2-alkyl-N-carboxymethyl-N-hydroxyethyl imidazolinium betaine: 2.0% by weight
cocamidopropyl betaine solution: 1.0% by weight
polyquaternium-6: 2.0% by weight
glycerin: 2.0% by weight
cationic locust bean gum: 0.3% by weight
colorant: dosage
fragrance: dosage
purified water: balance

### [prescription example 2-6: composition for hair shampoo]

lauric acid taurine sodium salt: 2.0% by weight
sodium polyoxyethylene laurylether sulfate: 3.0% by weight
lauryldimethylamino acetic acid betaine: 1.0% by weight
cocamidopropyl betaine solution: 2.0% by weight
propyltrimoniumchloride acrylamide/dimethylacrylamide copolymer, purified water mixture: 0.2% by weight
sorbitol solution: 3.0% by weight
succinic acid: 0.04% by weight
camellia oil: 0.3% by weight
colorant: dosage
fragrance: dosage
purified water: balance

### [prescription example 2-7: composition for body shampoo]

glycerin: 10.0% by weight
dipropylene glycol: 5.0% by weight
lauric acid triethanolamine: 6.0% by weight
lauryldimethylamino acetic acid betaine: 2.0% by weight
chamomilla extract: dosage
edetate trisodium: dosage
antiseptic agent: dosage
colorant: dosage
fragrance: dosage
purified water: balance

### [prescription example 2-8: composition for face cleansing foam]

glycerin: 25.0% by weight
polyethylene glycol 1500: 3.0% by weight
sorbitol solution: 3.0% by weight
stearic acid: 0.05% by weight
lauric acid: 2.0% by weight
myristic acid: 3.0% by weight
coconut diethanolamide: 0.3% by weight
cocamidopropyl betaine solution: 2.0% by weight
potassium hydroxide: 1.8% by weight
melissa extract: 0.1% by weight
edetate trisodium: dosage
fragrance: dosage
purified water: balance

### [prescription example 2-9: composition for face cleansing foam]

glycerin: 10.0% by weight
dipropylene glycol: 5.0% by weight
1,3-butylene glycol: 5.0% by weight
polyethylene glycol 1500: 3.0% by weight
sorbitol solution: 20.0% by weight
lauric acid: 2.5% by weight
myristic acid: 0.5% by weight
N-methyltaurine sodium salt: 2.0% by weight
lauryldimethylamino acetic acid betaine: 1.0% by weight
rosa roxburghii fruit extract: 0.2% by weight
dipotassium glycyrrhizate: 0.05% by weight
edetate trisodium: dosage
fragrance: dosage
purified water: balance

### [prescription example 2-10: composition for hand soap]

propylene glycol: 6.0% by weight
lauric acid: 3.0% by weight
myristic acid: 1.0% by weight
disodium laureth sulfosuccinate: 1.0% by weight
coconut diethanolamide: 0.5% by weight
cocamidopropyl betaine solution: 2.0% by weight
triethanolamine: 2.5% by weight
sodium chloride: 0.05% by weight
edetate trisodium: dosage
fragrance: dosage
purified water: balance

### [prescription example 2-11: composition for hand soap]

propylene glycol: 10.0% by weight
polyoxyethylene lauryl ether: 1.0% by weight
sodium C14-16 olefin sulfonate solution: 6.0% by weight
malic acid: 0.1% by weight
eucalyptus oil: 0.05% by weight
sodium benzoate: 0.1% by weight
benzalkonium chloride solution: 0.1% by weight
edetate trisodium: dosage
purified water: balance

### [Note]

<a composition for foam cleansing agent and a method of cleansing /foam for cleansing and method of generating the foam and a method of cleansing hair>

An embodiment is related to a composition for foam cleansing agent and a method of cleansing.

An embodiment relates to foam for cleansing, a method of generating the foam and a method of cleansing hair.

An object of an embodiment is to provide a composition for foam cleansing agent and a method of cleansing using the composition for foam cleansing agent capable of generating a foam cleansing agent with a higher detergency and sensation even when the content of the surfactant is small.

Another object of an embodiment is to provide fine and creamy foam for cleansing.

Another object of an embodiment is to provide a method of generating foam for cleansing capable of efficiently and safely generating fine and creamy foam for cleansing using a cleansing composition with a lower content of the surfactant compared with a conventional composition.

Further another object of an embodiment is to provide a method of cleansing hair capable of reducing a load to a person who performs a cleansing hair operation.

An embodiment (1-1) is a composition for foam cleansing agent including water and a surfactant used by generating bubbles by mixing with air such that the foam viscosity of the foam cleansing agent at 30°C at 10 seconds after the foam is generated and the average diameter of bubbles of the foam cleansing agent at 30 seconds after the foam is generated are more than or equal to 40 mPa•s and less than or equal to 100 mPa•s and more than or equal to 10µm and less than or equal to 100µm, respectively, where the content of the surfactant is more than or equal to 0.4% by weight and less than or equal to 12% by weight and the viscosity at 30°C is more than or equal to 5 mPa•s and less than or equal to 1500 mPa•s.

Another embodiment (1-2) is a composition for foam cleansing agent of the embodiment (1-1), wherein the foam viscosity of the foam cleansing agent at 30°C at 40 seconds after the foam is generated and the average diameter of bubbles of the foam cleansing agent at 60 seconds after the foam is generated are more than or equal to 35 mPa•s and less than or equal to 150µm, respectively.

Another embodiment (1-3) is a composition for foam cleansing agent of the embodiment (1-1) or (1-2), wherein the surfactant includes an amphoteric surfactant and an anionic surfactant.

Another embodiment (1-4) is a composition for foam cleansing agent of the embodiment (1-1) or (1-2), wherein the surfactant is an amphoteric surfactant and further includes a cationic polymer.

Another embodiment (1-5) is a composition for foam cleansing agent of the embodiment (1-4), wherein the cationic polymer is a cationic cellulose.

Another embodiment (1-6) is s composition for foam cleansing agent of the embodiment (1-4) or (1-5), wherein the content of the cationic polymer is more than or equal to 0.1% by weight and less than or equal to 1% by weight.

Another embodiment (1-7) is s composition for foam cleansing agent of the embodiment (1-1) or (1-2), wherein the surfactant is an amphoteric surfactant, and further includes an anionic polymer.

Another embodiment (1-8) is s composition for foam cleansing agent of the embodiment (1-7), wherein the anionic polymer is a xanthan gum.

Another embodiment (1-9) is a composition for foam cleansing agent of the embodiment (1-7) or (1-8), wherein the content of the anionic polymer is more than or equal to 0.01% by weight and less than or equal to 0.5% by weight.

Another embodiment (1-10) is a composition for foam cleansing agent of any one of the embodiments (1-1) to (1-9), wherein the foam is generated by a foam generating apparatus including a screw rotated by a motor.

Another embodiment (1-11) is a method of cleansing, including a step of generating the foam cleansing agent by generating the foam in the composition for foam cleansing agent of any one of the embodiments (1-1) to (1-10), and a step of cleansing using the foam cleansing agent.

Another embodiment (2-1) is foam for cleansing generated by mixing a cleansing composition with air, wherein the content of a surfactant is more than and equal to 0.4% by weight and less than or equal to 12% by weight, the foam viscosity at immediately after the foam is generated is more than or equal to 40 mPa•s and less than or equal to 100 mPa•s and the average diameter of the bubbles included in the foam at 30 seconds after the foam is generated is more than or equal to 10µm and less than or equal to 100µm.

Another embodiment (2-2) is foam for cleansing of the embodiment (2-1), wherein the viscosity of the cleansing composition at 30°C is more than or equal to 5 mPa•s and less than or equal to 1500 mPa•s.

Another embodiment (2-3) is foam for cleansing of the embodiment (2-1) or (2-2), wherein the difference between the viscosity at immediately after (10 seconds after) the foam is generated by mixing the cleansing composition with air and the viscosity at 40 seconds after the foam is generated is more than or equal to 7 mPa•s and less than or equal to 23 mPa•s, as well as the average diameter of the bubbles included in the foam at 60 seconds after the foam is generated is less than or equal to 150µm.

Another embodiment (2-4) is foam for cleansing of any one of the embodiments (2-1) to (2-3), wherein the cleansing composition is a shampoo.

An embodiment (2-5) is the foam for cleansing of any one of the embodiments (2-1) to (2-3), wherein the cleansing composition is a body shampoo.

Another embodiment (2-6) is foam for cleansing of any one of the embodiments (2-1) to (2-3), wherein the cleansing composition is a face-wash.

An embodiment (2-7) is the foam for cleansing of any one of the embodiments (2-1) to (2-3), wherein the cleansing composition is a hand soap.

Another embodiment (2-8) is a method of generating the foam for cleansing of any one of the embodiment (2-1) to (2-7) including a step of generating the foam for cleansing using a foam generating apparatus including a screw rotated by a motor and mixing the cleansing composition and air by the rotation of the screw.

Another embodiment (2-9) is a method of generating the foam for cleansing of the embodiment (2-8), wherein the foam generating apparatus includes a switch for driving and terminating the motor, the motor is driven when a user operates the switch to discharge the foam for cleansing, and the motor is terminated when the user releases the operation of the switch to terminate the discharging of the foam for cleansing.

Another embodiment (2-10) is a method of cleansing hair by placing the foam generating apparatus capable of generating the foam for cleansing of any one of the embodiments (2-1) to (2-3) at a hair cleansing counter at which cleansing of hair is performed, and cleansing hair using the foam for cleansing generated by the foam generating apparatus.

According to an embodiment, a composition for foam cleansing agent and a method of cleansing hair using the composition for foam cleansing agent capable of generating a foam cleansing agent which is good in detergency and sensation, even when the content of the surfactant is small.

According to an embodiment, fine and creamy foam for cleansing with a good foam persistency can be obtained. Further, the surfactant can be reduced to prevent a trouble to the hand of a user even when it is used in a service. Further, as the surfactant is reduced cost of the foam for cleansing can be reduced.

The present invention is not limited to the specifically disclosed embodiments, and variations and modifications may be made without departing from the scope of the present invention.

The present application is based on Japanese Priority Applications No. 2009-278766 filed on December 8, 2009, and No. 2009-280414 filed on December 10, 2009, the entire contents of which are hereby incorporated herein by reference.

### DESCRIPTION OF MARKS AND NUMERALS

- 101: foam generating apparatus
- 102: body portion
- 103: hand inserting portion
- 104: operation portion
- 105: tank
- 106: cover member
- 107: motor
- 108: foam generation room
- 109: screw
- 110: open valve
- 111: coil spring
- 112: shaft
- 113: valve body
- 114: admission port
- 115: switch
- 116: discharge port
- A1: composition for foam cleansing agent
- A1': foam cleansing agent
- 201: foam generating apparatus
- 202: body portion
- 203: hand inserting portion
- 204: operation portion
- 205: liquid tank
- 207: motor
- 208: foam generation room
- 209: screw
- 210: open valve
- 214: air admission port
- 215: switch
- 216: foam discharge port
- 220: beauty salon
- 221: shampoo counter
- 223: container for cosmetics
- A2: foam for cleansing
- B2: original liquid of cleansing composition

## Claims

1. A cleansing composition comprising:
water; and
a surfactant, the content of the surfactant in the cleansing composition being more than or equal to 0.4% by weight and less than or equal to 12% by weight, the viscosity of the cleansing composition at 30°C being more than or equal to 5 mPa•s and less than or equal to 1500 mPa•s, the foam viscosity of foam at 30°C at 10 seconds after the foam is generated by mixing the cleansing composition with air being more than or equal to 40 mPa•s and less than or equal to 100 mPa•s, and the average diameter of bubbles included in the foam at 30 seconds after the foam is generated by mixing the cleansing composition with air being more than or equal to 10µm and less than or equal to 100µm.

2. The cleansing composition according to claim 1,
wherein the foam viscosity of the foam at 30°C at 40 seconds after the foam is generated by mixing the cleansing composition with air is more than or equal to 35 mPa•s, as well as
the average diameter of the bubbles included in the foam at 60 seconds after the foam is generated by mixing the cleansing composition with air is less than or equal to 150µm.

3. The cleansing composition according to claim 1,
wherein the difference between the foam viscosity of the foam generated by mixing the cleansing composition with air at 10 seconds after the foam is generated and the foam viscosity of the foam generated by mixing the cleansing composition with air at 40 seconds after the foam is generated is more than or equal to 7 mPa·s and less than or equal to 23 mPa·s, as well as
the average diameter of the bubbles included in the foam generated by mixing the cleansing composition with air at 60 seconds after the foam is generated is less than or equal to 150µm.

4. The cleansing composition according to claim 1,
wherein the surfactant includes an amphoteric surfactant and an anionic surfactant.

5. The cleansing composition according to claim 1,
wherein the surfactant is an amphoteric surfactant, and the cleansing composition further comprises a cationic polymer.

6. The cleansing composition according to claim 5,
wherein the cationic polymer is a cationic cellulose.

7. The cleansing composition according to claim 5,
wherein the content of the cationic polymer in the cleansing composition is more than or equal to 0.1% by weight and less than or equal to 1% by weight.

8. The cleansing composition according to claim 1,
wherein the surfactant is an amphoteric surfactant, and the cleansing composition further comprises an anionic polymer.

9. The cleansing composition according to claim 8,
wherein the anionic polymer is a xanthan gum.

10. The cleansing composition according to claim 8,
wherein the content of the anionic polymer in the cleansing composition is more than or equal to 0.01% by weight and less than or equal to 0.5% by weight.

11. The cleansing composition according to claim 1,
wherein the cleansing composition is selected from a group including a shampoo, a body shampoo, a face-wash, and a hand soap.

12. A method of generating foam by mixing the cleansing composition according to claim 1 with air, comprising:
supplying the cleansing composition to a screw rotated by a motor; and
mixing the cleansing composition with air by rotating the screw.

13. The method of generating foam according to claim 12, further comprising controlling starting and terminating generating foam by switching driving and terminating of the motor.

14. Foam generated by mixing the cleansing composition according to claim 1 with air.

15. A method of cleansing hair by using the foam according to claim 14.
